# FASCICULE DE BREVET EUROPEEN

(11) **EP 3 914 581 B1**
(45) Date de publication et mention de la délivrance du brevet: **05.10.2022**
(21) Numéro de dépôt: 20706572.3
(22) Date de dépôt: 21.01.2020
(51) Int. Cl.: C07C 67/08, C07C 67/54, C07C 69/54

(54) **PROCEDE DE PURIFICATION D'ESTERS (METH)ACRYLIQUES A L'AIDE D'UNE COLONNE A PARTITION**
VERFAHREN ZUR REINIGUNG VON (METH)ACRYLSÄUREESTERN UNTER VERWENDUNG EINER TRENNWANDKOLONNE
METHOD FOR PURIFYING (METH)ACRYLIC ESTERS USING A DIVIDED WALL COLUMN

(30) Priorité: 22.01.2019 FR 1900541
(43) Date de publication de la demande: 01.12.2021
(73) Titulaire: ARKEMA FRANCE, 92700 Colombes (FR)
(72) Inventeur: TRETJAK, Serge, 57501 Saint Avold cedex (FR); CABON, Yves, 57501 Saint Avold cedex (FR); LEVRAY, André, 57501 Saint Avold cedex (FR); HILPERT, Camille, 57501 Saint Avold cedex (FR); MORELIERE, Anne, 57501 Saint Avold cedex (FR)
(74) Mandataire: Arkema Patent
(86) Numéro de dépôt international: PCT/FR2020/050075
(87) Numéro de publication internationale: WO 2020/152415

(56) Documents cités:
- WO-A1-2018/114429
- DEJANOVIC I ET AL: "Dividing wall column-A breakthrough towards sustainable distilling", CHEMICAL ENGINEERING AND PROCESSING, ELSEVIER SEQUOIA, LAUSANNE, CH, vol. 49, no. 6, 1 juin 2010 (2010-06-01), pages 559-580, XP027138843, ISSN: 0255-2701 [extrait le 2010-04-12] cité dans la demande
- ASPRION N ET AL: "Dividing wall columns: Fundamentals and recent advances", CHEMICAL ENGINEERING AND PROCESSING, ELSEVIER SEQUOIA, LAUSANNE, CH, vol. 49, no. 2, 1 février 2010 (2010-02-01), pages 139-146, XP026938678, ISSN: 0255-2701, DOI: 10.1016/J.CEP.2010.01.013 [extrait le 2010-01-25] cité dans la demande

## Description

### DOMAINE TECHNIQUE

La présente invention concerne la production d'esters (méth)acryliques selon un procédé par estérification directe, et en particulier la purification d'un mélange réactionnel brut comprenant un ester (méth)acrylique en C₄-C₁₂ à l'aide d'une colonne à partition mise en oeuvre dans une configuration particulière.

Cette configuration conduit à une simplification du procédé de purification et garantit un produit d'une pureté très élevée, indépendamment des réactions de rétro-craquage des sous-produits lourds, susceptibles de se produire au cours de la purification du produit recherché.

L'invention a trait également à un procédé de production d'ester (méth)acrylique en C₄- C₁₂, comprenant ce procédé de récupération/purification.

### ARRIERE-PLAN TECHNIQUE ET PROBLEME TECHNIQUE

La synthèse d'esters (méth)acryliques en C₄-C₁₂ peut être effectuée par réaction d'estérification directe de l'acide (méth)acrylique avec l'alcool C₄-C₁₂ correspondant. Cette réaction est généralement réalisée en présence d'un catalyseur homogène, notamment un catalyseur acide, tel que l'acide sulfurique ou l'acide para toluène sulfonique.

L'estérification est une réaction équilibrée avec génération d'eau, l'élimination de l'eau pour déplacer l'équilibre dans le sens de la production de l'ester (méth)acrylique pouvant être assurée sous forme d'azéotrope à l'aide d'un excès de l'alcool estérifiant.

Des réactions secondaires au cours de la synthèse produisent des impuretés, généralement des sous-produits de point d'ébullition élevé ou proche du point d'ébullition de l'ester recherché, généralement sous la forme de composés dénommés adduits de Michael. Il est nécessaire d'éliminer ces composés en vue d'obtenir l'ester (méth)acrylique avec une pureté élevée satisfaisant aux exigences techniques liées à son utilisation finale.

A ces fins, on procède généralement à un processus de séparation/purification comportant un ensemble de distillations, d'extractions et /ou décantations qui sont des opérations coûteuses d'un point de vue énergétique, mise au mille et bien entendu en termes d'investissement et frais de construction pour un atelier industriel.

Les opérations de purification sont complexes à mettre en oeuvre, notamment du fait de la présence de mélanges azéotropiques, mais aussi en raison de la présence d'un catalyseur homogène, généralement acide, dans le milieu réactionnel à purifier. Dans le cas par exemple d'une catalyse par l'acide sulfurique, la réaction d'estérification génère un mélange réactionnel comprenant l'ester recherché, les réactifs - alcool et acide - résiduels, et l'acide sulfurique sous forme de sulfate acide d'alkyle. Le mélange réactionnel est alors soumis à une neutralisation, par exemple par addition d'une solution aqueuse de base, suivie du traitement de la phase aqueuse et de la phase organique séparées après décantation, comme décrit par exemple dans les documents EP 609 127 et EP 1 247 793. Cependant, en cas de neutralisation incomplète ou de lavage incomplet de la phase organique séparée, l'acidité résiduelle peut induire des problèmes de dégradation de l'ester acrylique formé ou le craquage des adduits de Michael au cours des étapes de purification de la phase organique. Dans ces conditions, une pureté élevée pour l'ester (méth)acrylique devient difficile à atteindre.

Le brevet US 6,320,070 décrit des conditions opératoires de synthèse d'esters (méth)acryliques par estérification directe catalysée par l'acide sulfurique, minimisant la formation d'adduits de Michael et incluant l'extraction d'une phase aqueuse concentrée en catalyseur qui est réintroduite à la réaction. Cependant, ce document ne mentionne pas les problèmes liés à la présence éventuelle d'acidité résiduelle dans le mélange soumis aux étapes de purification.

De façon générale, les documents de l'art antérieur associent dans les procédés d'estérification directe en présence d'un catalyseur homogène tel que l'acide sulfurique, une séparation et une neutralisation préalable avec une base comme de la lessive de soude, une décantation suivie d'un lavage de la phase organique avant purification à l'aide d'une colonne d'étêtage (distillation des composés légers) et une colonne d'équeutage (séparation des composés lourds). Dans tous les cas, la purification ainsi réalisée ne permet pas d'obtenir avec un fort taux de récupération, un produit final de haute pureté dans des conditions économiques.

Avec le développement des colonnes de distillation à partition (connues sous la dénomination DWC- Divided wall column), des procédés de purification simplifiés sont maintenant proposés. Cette technologie est basée sur une colonne de distillation comprenant une paroi interne de séparation ce qui permet de combiner le fonctionnement de deux colonnes traditionnellement en série dans un seul appareillage, en mettant en oeuvre un rebouilleur et un condenseur unique.

A titre d'exemple, la demande de brevet EP 2 659 943 décrit une configuration d'une colonne à partition et son fonctionnement dans un procédé de production d'acrylate de 2-éthylhexyle de haute pureté. Bien que cette colonne soit complexe à fabriquer et à opérer, elle présente l'avantage de réduire le coût d'équipement et la consommation d'énergie du processus de purification, comparativement à une installation conventionnelle comprenant deux colonnes de distillation. Le procédé de purification décrit dans ce document ne fait pas mention des problèmes liés à la séparation préalable du catalyseur dont la présence résiduelle peut engendrer des réactions de rétrogradation dans la colonne à partition. De plus, la question de la stabilisation nécessaire à son bon fonctionnement n'est pas abordée.

La demande de brevet JP 2005-239564 décrit également l'utilisation d'une colonne à partition dans un procédé de synthèse d'esters (méth)acryliques, exemplifiée dans le cas de la synthèse de méthacrylate de butyle par réaction de transestérification entre le méthacrylate de méthyle et le butanol. Dans ce procédé, un dévésiculeur est associé à la colonne à partition pour éviter l'entraînement de gouttelettes de stabilisants dans le soutirage latéral et contrôler la quantité de stabilisants dans le produit purifié. La colonne à partition permet d'effectuer la séparation de l'ester visé avec les produits lourds et les produits plus légers. Le procédé de purification décrit dans ce document est applicable à la production de (méth)acrylates d'alkyle en C₁-C₄ par estérification directe. Cependant, il ne résout pas la question de la séparation préalable du catalyseur lorsque les esters sont sensibles à des réactions de rétrogradation, en particulier lorsque la présence du catalyseur dans la colonne à partition risque d'engendrer des réactions de craquage conduisant à la formation de composés polluant le produit purifié soutiré latéralement.

Dans le document WO 2018/114429, une colonne à partition comportant une partie inférieure commune reliée à un rebouilleur unique est utilisée pour purifier l'acrylate de 2-éthyl hexyle ou l'acrylate de 2-propyl heptyle. Là encore, ce type de configuration ne pose pas le problème des réactions de rétro-craquage susceptibles de se produire dans le bouilleur de cette colonne en générant des impuretés légères comme l'alcool estérifiant et par voie de conséquence, en affectant la pureté du produit final.

La technologie des colonnes à partition en général est connue, et décrite par exemple par Asprion N et al, « Dividing wall columns : Fundamentals and recent advances », Chemical Engineering and processing, vol 49 (2010) pages 139-146 ; ou par Dejanovic I et al, « Dividing wall column - A breakthrough towards sustainable distilling », Chemical Engineering and processing, vol 49 (2010) pages 559-580.

Cependant, les différentes configurations décrites concernent uniquement le gain énergétique attendu, et l'état de la technique dans son ensemble ne suggère nullement le type de configuration adapté à la purification de mélanges réactionnels (méth)acryliques bruts issus d'une réaction d'estérification en catalyse homogène, notamment lorsque le milieu à purifier comporte des adduits de Michael et un catalyseur acide tel que l'acide sulfurique.

Il subsiste encore un besoin d'améliorer la purification des esters (méth)acryliques en C₄-C₁₂, comme par exemple l'acrylate de 2-éthyl hexyle ou l'acrylate de 2-octyle, afin d'obtenir un produit d'une pureté très élevée, indépendamment des réactions de rétro-craquage des sous-produits lourds qui se produisent inévitablement au cours du processus de purification.

L'objectif de la présente invention répond à ce besoin en fournissant un procédé de récupération d'un ester (méth)acrylique en C₄-C₁₂ purifié à l'aide d'un système de purification comprenant une colonne à partition mise en œuvre dans une configuration particulière, qui permet d'éviter le risque de pollution de l'ester produit fini par les réactions de rétrogradation en présence de catalyseur résiduel dans la colonne.

La présente invention fournit ainsi un procédé simplifié pour récupérer un ester (méth)acrylique en C₄-C₁₂ de haute pureté à partir d'un mélange réactionnel brut résultant de la réaction d'estérification directe de l'acide (méth)acrylique par l'alcool correspondant tout en optimisant le coût d'investissement et l'énergie à mettre en oeuvre dans l'installation.

### RESUME DE l'INVENTION

L'invention a pour objet un procédé de récupération d'un ester (méth)acrylique en C₄-C₁₂ purifié, à partir d'un mélange réactionnel brut obtenu par estérification directe de l'acide (méth)acrylique avec l'alcool en C₄-C₁₂ correspondant, ledit procédé étant caractérisé en ce qu'il est mis en oeuvre à l'aide d'un système de purification comprenant une colonne à partition équipée d'une paroi séparatrice non jointive avec le dôme supérieur de la colonne en partie haute et jointive avec le fond de la colonne en partie basse, et associée en tête à un condenseur unique et en pied à deux bouilleurs, ladite colonne à partition comprenant une section commune de rectification au-dessus de la paroi, une section de préfractionnement comportant l'alimentation de la colonne et une section de soutirage, séparée de la section de préfractionnement par la paroi, comprenant le soutirage de l'ester purifié.

Selon un mode de réalisation, un flux gazeux est extrait en tête de la section de rectification et recyclé après condensation au moins en partie dans le réacteur d'estérification.

Selon un mode de réalisation, un flux est soutiré en pied de la section de préfractionnement et recyclé au moins en partie dans le réacteur d'estérification.

Selon un mode de réalisation, un flux est soutiré en pied de la section de soutirage et recyclé au moins en partie dans la section de préfractionnement de la colonne à partition.

Selon un mode de réalisation, un flux d'ester (méth)acrylique purifié est soutiré latéralement de la section de soutirage en un point situé au-dessus du soutirage de pied de ladite section de soutirage.

Selon un mode de réalisation, la réaction d'estérification directe est effectuée en présence d'un catalyseur homogène, notamment un catalyseur acide, tel que l'acide sulfurique ou l'acide para toluène sulfonique.

Selon un mode de réalisation, le mélange réactionnel brut est soumis au préalable à une neutralisation avec une base suivie d'une décantation et d'un lavage de la phase organique avant purification.

Selon un mode de réalisation, le mélange réactionnel brut soumis au procédé de récupération de l'ester (méth)acrylique purifié, comprend au moins en partie le catalyseur mis en oeuvre pour la réaction d'estérification.

Selon un mode de réalisation, la stabilisation du système de purification est réalisée à l'aide d'un inhibiteur de polymérisation unique, de préférence injecté au niveau du condenseur de tête, l'ester (méth)acrylique purifié étant soutiré latéralement de la colonne à partition sous forme d'un flux liquide ou gazeux déjà stabilisé.

Selon un mode de réalisation, la stabilisation du système de purification est réalisée à l'aide d'un premier inhibiteur de polymérisation, de préférence injecté au niveau du condenseur de tête, l'ester (méth)acrylique purifié étant soutiré latéralement de la colonne à partition sous forme d'un flux gazeux qui, après condensation, est ensuite stabilisé avec un inhibiteur de polymérisation différent du premier inhibiteur.

Selon l'invention, l'inhibiteur de polymérisation utilisé pour stabiliser l'ester recherché peut être introduit dans le système de purification comme inhibiteur de polymérisation unique, il s'ensuit une simplification et une constance de la stabilisation. En alternative, un inhibiteur de polymérisation moins onéreux peut être utilisé pour stabiliser la colonne à partition, et l'ester purifié est ensuite stabilisé avec un autre composé plus adapté pour stabiliser le produit fini en vue de son stockage et utilisation ultérieure. Dans ce cas, le coût lié aux inhibiteurs de polymérisation peut être fortement réduit.

Selon l'invention, l'alcool estérifiant est un alcool aliphatique primaire ou secondaire, à chaine alkyle linéaire ou ramifiée comportant de 4 à 12 atomes de carbone, de préférence de 5 à 10 atomes de carbone.

Comme exemples d'alcool, on peut citer le 2-éthyl hexanol, le 2-octanol, ou le 2-propyl heptanol. De préférence, l'alcool est le 2-éthyl hexanol.

Le terme « (méth)acrylique » signifie acrylique ou méthacrylique ; le terme « (méth)acrylate » signifie acrylate ou méthacrylate.

De préférence, l'acide (méth)acrylique est l'acide acrylique.

Selon un mode de réalisation préféré, l'ester (méth)acrylique en C₄-C₁₂ purifié est un acrylate en C₄-C₁₂ purifié, plus préférentiellement l'acrylate de 2-éthyl hexyle ou l'acrylate de 2-octyle.

Le procédé de récupération selon l'invention conduit à un (méth)acrylate en C₄-C₁₂ de pureté supérieure à celle obtenue dans une installation conventionnelle comprenant au moins deux colonnes de séparation, et cela dans des conditions énergétiques plus économiques, grâce à des conditions de fonctionnement de la colonne à partition qui minimisent la dégradation thermique des composés thermosensibles présents dans le milieu à purifier.

Un autre objet de l'invention est l'utilisation d'un système de purification pour récupérer un ester (méth)acrylique en C₄-C₁₂ purifié, à partir d'un mélange réactionnel brut obtenu par estérification directe de l'acide (méth)acrylique avec l'alcool en C₄-C₁₂ correspondant, ledit système de purification comprenant une colonne à partition équipée d'une paroi séparatrice non jointive avec le dôme supérieur de la colonne en partie haute et jointive avec le fond de la colonne en partie basse, et associée en tête à un condenseur unique et en pied à deux bouilleurs, ladite colonne à partition comprenant une section commune de rectification au-dessus de la paroi, une section de préfractionnement comportant l'alimentation de la colonne et une section de soutirage, séparée de la section de préfractionnement par la paroi, comprenant le soutirage de l'ester purifié.

Un autre objet de l'invention est un procédé de production d'un ester (méth)acrylique en C₄-C₁₂ de haute pureté, par estérification directe de l'acide (méth)acrylique avec l'alcool en C₄-C₁₂ correspondant, caractérisé en ce que le mélange réactionnel brut est soumis au procédé de récupération à l'aide du système de purification tel que défini ci-dessus.

Ainsi, l'invention permet d'atteindre les spécifications souhaitées en terme de pureté des esters (méth)acryliques dans des conditions économiques, à savoir une pureté supérieure à 99,6%, voire supérieure à 99,8%.

### BREVE DESCRIPTION DES FIGURES

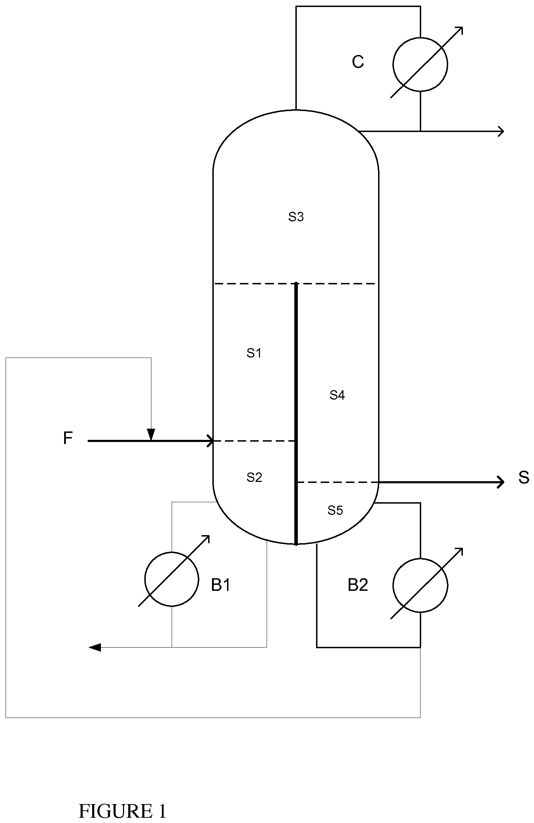

La Figure unique représente la configuration d'un système de purification comprenant une colonne à partition utilisable dans le procédé selon l'invention.

### EXPOSE DETAILLE DE L'INVENTION

L'invention est maintenant décrite plus en détail et de façon non limitative dans la description qui suit.

En référence à la Figure, la colonne à partition utilisée dans le procédé selon l'invention comporte une paroi (ou cloison) verticale partielle P, placée à l'intérieur de la colonne définissant ainsi trois zones distinctes : une zone supérieure, dénommée section de rectification, une zone centrale comprenant deux zones de part et d'autre de la cloison et s'étendant jusqu'au bas de la colonne.

Selon un mode de réalisation, la paroi peut être en partie diagonale. La paroi peut être plane ou cylindrique de sorte que les espaces séparés par la paroi peuvent être disposés sous forme concentrique.

La paroi telle que mise en place ne sépare pas forcément la zone centrale en deux zones égales, il peut en effet être avantageux dans certains modes de réalisation d'avoir des zones inégales afin de minimiser la perte de charge ou la tendance à l'engorgement selon la nature ou l'intensité des flux circulant dans la colonne.

La hauteur de la paroi peut représenter de 30 à 70 % de la hauteur de la colonne.

La zone centrale est constituée de deux zones de part et d'autre de la paroi, dont l'une représente une section dite de préfractionnement et l'autre une section de soutirage du produit pur.

Par souci de simplification dans la suite de l'exposé de l'invention, par « section de préfractionnement », on entend la section de la colonne à paroi séparatrice qui est alimentée par le flux d'ester (méth)acrylique à purifier, l'alimentation ne se faisant que d'un seul côté de la paroi ; et par « section de soutirage », on entend la section de la colonne de l'autre côté de la paroi séparatrice d'où est extrait latéralement le flux d'ester (méth)acrylique purifié.

La section de préfractionnement associée à un bouilleur B1, comporte l'alimentation F de la colonne, séparant ainsi une section S1 au-dessus de l'alimentation et une section S2 au-dessous de l'alimentation. La section de préfractionnement a pour effet de concentrer les produits les plus volatils dit composés légers, en tête de la colonne, et de concentrer les produits les moins volatils dits composés lourds en pied de la colonne. C'est en particulier dans cette section de préfractionnement associée au bouilleur B1 que l'on retrouve en pied de celle-ci une grande partie des inhibiteurs de polymérisation ainsi que les impuretés lourdes. Ce pied de préfractionnement peut être valorisé en recyclant tout ou partie de celui-ci, dans le réacteur d'estérification, éventuellement après passage sur un évaporateur à film.

Selon un mode de réalisation, l'alimentation se situe à la moitié inférieure de la section de préfractionnement, comprenant S1+S2, de préférence au tiers inférieur, par exemple au plateau 4.

La section de soutirage comporte une sortie latérale afin de soutirer l'ester purifié S, la sortie latérale divisant la section de soutirage en deux sections S4 et S5. Le soutirage de l'ester purifié peut être réalisé sous forme d'un flux liquide ou d'un flux gazeux, de préférence on soutire un flux gazeux. Dans cette section, les composés légers ainsi que l'ester sont envoyés en tête de la colonne et les composés lourds sont envoyés en pied de la colonne. Un flux de pied comprenant essentiellement des composés lourds et des inhibiteurs de polymérisation et un peu d'ester produit, est soutiré de la section de soutirage associée à un bouilleur B2, et est recyclé avantageusement, au moins en partie, dans la section de préfractionnement, de préférence au niveau de l'alimentation F, ou en un point situé au-dessus ou en-dessous de l'alimentation. Le recyclage éventuel du pied de la section de soutirage permet de minimiser les pertes en ester (méth)acrylique.

Selon un mode de réalisation, le point de soutirage latéral se situe dans la moitié inférieure de la section de soutirage, de préférence au quart inférieur.

Au-dessus de la paroi en tête de la colonne à partition se trouve une zone commune, dite section de rectification S3 qui permet de séparer les composés légers qui sont extraits, puis condensés au moins en partie, dans le condenseur C associé à la colonne. Ce produit condensé est renvoyé en partie comme reflux dans la section S3, l'autre partie étant envoyé avantageusement au moins en partie à l'entrée du réacteur puisqu'il est constitué principalement de réactifs non réagis et un peu d'ester formé.

Le reflux liquide sur les sections de préfractionnement et de soutirage (non représenté) est assuré par un moyen de collecte permettant de répartir de façon contrôlée le liquide issu du bas de la section de rectification vers les sections de préfractionnement et de soutirage.

La fraction massique de liquide retournant vers la section S1 est généralement comprise entre 20 et 50%.

Le pied de la section de soutirage comportant également un bouilleur B2 est recyclé de façon avantageuse à l'alimentation de la colonne à partition permettant ainsi de recycler et minimiser les pertes en ester en pied de cette section de soutirage.

Un certain nombre de paramètres caractérisent le design et le fonctionnement de la colonne à partition. Il s'agit principalement du nombre d'étages théoriques dans chaque section de la colonne à partition, en particulier les nombres N1, N2, N3, N4, N5 correspondant respectivement au nombre d'étages de chacune des sections S1 à S5 décrites précédemment, du taux de reflux de la colonne, du rapport de flux liquide provenant de la section de rectification de chaque côté de la paroi, du rapport de flux gazeux provenant de la section de rebouillage de chaque côté de la cloison, du positionnement du point d'alimentation F ou du point de soutirage latéral S du produit pur.

Ces différents paramètres peuvent être déterminés à partir de méthodes connues par l'homme de l'art de façon à ce que l'ester (méth)acrylique soit produit avec une pureté répondant aux spécifications désirées.

La colonne à partition et les internes présents sont choisis pour obtenir le nombre d'étages théoriques nécessaires dans chaque section. On pourra utiliser comme internes des plateaux, du garnissage ordonné comme du garnissage structuré ou du garnissage en vrac.

Selon un mode de réalisation, le nombre d'étages théoriques de la section de préfractionnement S1+S2 est compris entre 1 et 15, et l'alimentation de la colonne est de préférence placée au dernier tiers inférieur environ de cette section.

Selon un mode de réalisation, le nombre d'étages théoriques de la section de soutirage S4+S5 est compris entre 2 et 15, et le point de soutirage de l'ester purifié est de préférence placé au dernier quart inférieur environ de cette section.

Selon un mode de réalisation, le nombre d'étages théoriques de la section de rectification S3 est compris entre 5 et 15.

La colonne peut opérer sous vide, afin de minimiser l'exposition thermique des composés thermosensibles au sein de la colonne. Avantageusement, la colonne fonctionne sous un vide allant de 10 à 100 mm de Hg.

Avantageusement la température de fonctionnement est comprise entre 50°C et 160°C.

Les internes utilisés pour la colonne peuvent être des plateaux à clapets ou des plateaux perforés à déversoir, ou des plateaux à courants croisés comme les Dual Flow les Ripple Trays, les Turbo Grid Shell, ou du garnissage ordonné comme du garnissage structuré tel le Mellapack 250X de Sulzer.

Le procédé selon l'invention a pour objectif de récupérer l'ester (méth)acrylique avec une pureté supérieure à 99,6%, de préférence supérieure à 99,8% à partir d'un mélange réactionnel brut, obtenu selon les procédés connus d'estérification directe, notamment selon les procédés décrits dans les documents EP 609 127 et EP 1 247 793.

Les conditions de la réaction d'estérification sont celles connues par l'homme de l'art, et peuvent être mises en œuvre selon un procédé de type continu, semi-continu ou discontinu, de préférence selon un procédé en continu.

Comme catalyseur d'estérification, on utilise généralement un catalyseur homogène, le plus souvent acide, tel qu'un acide organique sulfonique, comme l'acide méthane sulfonique, l'acide para-toluène sulfonique, l'acide benzène sulfonique, l'acide dodécyl sulfonique, l'acide xylène sulfonique, ou leurs mélanges, ou l'acide sulfurique. De préférence, on utilise l'acide sulfurique.

Le mélange réactionnel comprend l'ester recherché, les réactifs - alcool et acide - résiduels (composés légers), et l'acide sulfurique sous forme de sulfate acide d'alkyle dans le cas d'une catalyse acide sulfurique, ainsi que des composés lourds formés au titre de réactions secondaires.

Les composés dits lourds proviennent de réactions d'addition de Michael (adduits de Michael). Dans le cas de la synthèse de l'acrylate de 2-éthyl hexyle, il s'agit en particulier de 2-éthylhexylpropionate de 2-éthyle hexyle (OPO), de Béta hydroxypropionate de 2-éthyl hexyle ou propioxypropionate de 2-éthyl hexyle, et d'autres produits à point d'ébullition élevé.

Le mélange réactionnel brut formé dans le réacteur d'estérification peut être traité directement selon le procédé selon l'invention.

Selon une alternative préférée, le mélange réactionnel peut être soumis à une neutralisation, par exemple par addition d'une solution aqueuse de base telle que la soude, suivie d'une décantation séparant une phase aqueuse, et une phase organique. La phase aqueuse permet de séparer les sels formés après l'étape de neutralisation. La phase organique, après lavage éventuel à l'eau dans une colonne d'extraction pour éliminer les traces de sels, est ensuite envoyée dans le système de purification selon l'invention.

Dans ces conditions, la phase organique peut présenter une acidité résiduelle capable de catalyser la dégradation des adduits de Michael au cours du processus de purification.

La configuration de la colonne à partition mise en oeuvre dans le procédé selon l'invention permet d'éviter alors le risque de pollution du produit soutiré latéralement.

Hormis des conditions opératoires adaptées pour la réaction d'estérification minimisant la formation des composés lourds et optimisant le rendement de la réaction, il est nécessaire d'introduire des inhibiteurs de polymérisation (dénommés aussi stabilisants) non seulement au cours de la réaction, mais également au cours de la purification du mélange réactionnel brut sortant du réacteur d'estérification.

Comme inhibiteurs de polymérisation utilisables, on peut citer par exemple la phénothiazine (PTZ), l'hydroquinone (HQ), l'éther mono méthylique d'hydroquinone (EMHQ), le diterbutyl para-crésol (BHT), la paraphénylène diamine, le TEMPO (2,2,6,6-tétraméthyl-1-piperidinyloxy), le di-tertiobutylcatéchol, ou les dérivés du TEMPO, tel que le OH-TEMPO, seuls ou leurs mélanges en toutes proportions.

Avantageusement, on introduit de 100 à 5000 ppm d'inhibiteur de polymérisation lors de la purification du mélange réactionnel dans le système de purification selon le procédé de l'invention.

Pour rendre les inhibiteurs plus efficaces il convient d'injecter en pied de colonne de l'oxygène, de l'air ou de l'air dit appauvri à 7% O₂.De façon préférée, la quantité d'oxygène injectée correspond à une teneur de 0,2% à 0,5% rapportée à la quantité de vapeur organique dans la colonne.

Selon un premier mode de réalisation, la stabilisation du système de purification est réalisée à l'aide d'un inhibiteur de polymérisation unique, de préférence injecté au niveau du condenseur de tête, l'ester (méth)acrylique purifié étant soutiré latéralement de la colonne à partition sous forme d'un flux liquide ou gazeux stabilisé.

Selon ce mode de réalisation, on préfère utiliser l'éther mono méthylique d'hydroquinone comme stabilisant.

Selon un second mode de réalisation, on utilise un premier inhibiteur de polymérisation, injecté au niveau du condenseur de tête, pour limiter les réactions secondaires de polymérisation dans la colonne à partition, et on soutire latéralement l'ester (méth)acrylique purifié sous forme d'un flux gazeux qui, après condensation, est stabilisé avec un inhibiteur de polymérisation différent du précédent injecté dans le condenseur de tête. Selon ce mode de réalisation, il est possible d'utiliser un premier inhibiteur nettement moins cher et s'affranchir de sa présence dans le produit purifié en effectuant un soutirage en phase gazeuse, le premier inhibiteur de polymérisation restant dans le flux de sous-produits lourds séparé en pied de colonne. La phénothiazine convient comme premier inhibiteur de polymérisation car il permet également de stabiliser les flux organiques. L'ester (méth)acrylique purifié soutiré est alors stabilisé selon la pratique conventionnelle, par exemple à l'aide d'éther méthylique d'hydroquinone.

Par ester (méth)acrylique purifié, on entend un produit ayant une teneur en ester (méth)acrylique > 99,6 % en poids, de préférence > 99,8% en poids. De préférence, la teneur en impuretés lourdes est inférieure à 1000 ppm. De préférence, la teneur en alcool résiduel est inférieure à 1000 ppm, en particulier inférieure à 500 ppm.

L'invention fournit ainsi un procédé de production d'un ester (méth)acrylique en C₄-C₁₂ dans une installation compacte dont le coût d'investissement et de fonctionnement est réduit, et fournissant un produit de haute pureté avec un rendement optimisé.

Les exemples ci-après illustrent la présente invention sans toutefois en limiter la portée.

### PARTIE EXPERIMENTALE

Dans les exemples, les pourcentages sont indiqués en poids sauf indication contraire et les abréviations suivantes sont été utilisées :
A2EH : acrylate de 2-éthyl hexyle
AA : acide acrylique
PTZ : phénothiazine
HQ : hydroquinone

### Exemple 1 : Test de dégradation d'un produit de pied de colonne

Cet exemple met en évidence les phénomènes de rétro-craquage qui se produisent en pied d'une colonne de distillation DWC classique, c'est-à-dire comportant une paroi séparatrice non jointive en partie basse de la colonne et un seul rebouilleur associé en pied.

Le test de craquage a été réalisé dans un ballon d'un litre surmonté d'un condenseur pour assurer un fonctionnement à reflux total à pression atmosphérique.

Un mélange ayant une composition proche de la composition attendue en pied de la colonne de distillation au niveau du bouilleur B1 a été introduit dans le ballon et soumis à une température de 147°C et 156°C à P atm sur une durée de 40 minutes.

Un suivi chromatographique de la composition du mélange réactionnel a été réalisé et les résultats exprimés en pourcentages massiques sont rassemblés dans le tableau 1 ci-après,

**Tableau 1**

| | | 147 °C | | | 156 °C | |
|---|---|---|---|---|---|---|
| Temps, min | 0 | 20 | 40 | 0 | 20 | 40 |
| **2-éthyl hexanol** | **0,0390** | **0,066** | **0,0701** | **0,0390** | **0,0797** | **0,1120** |
| acétate de 2- éthyle hexyle | 0,0511 | 0,0493 | 0,0493 | 0,0511 | 0,0498 | 0,0498 |
| acrylate de 2-Ethyl 4-méthyl pentyle | 0,0554 | 0,0555 | 0,0563 | 0,0554 | 0,0550 | 0,0547 |
| acrylate en C8 | 0,0063 | 0,0110 | 0,0081 | 0,0063 | 0,0131 | 0,0105 |
| acrylate de 2-éthyl hexyle A2EH | 81,59 | 81,88 | 81,89 | 81,59 | 81,76 | 81,79 |
| butanoate de 2-éthyl hexyle | 0,0351 | 0,0346 | 0,0344 | 0,0351 | 0,0346 | 0,0347 |
| di octyl éther (3 isomères ) | 0,0206 | 0,0203 | 0,0196 | 0,0206 | 0,0206 | 0,0207 |
| di (2-éthyl hexyle) éther | 0,0277 | 0,0275 | 0,0275 | 0,0277 | 0,0275 | 0,0276 |
| béta (H₂O+A2EH) | 0,7885 | 0,8152 | 0,8140 | 0,7885 | 0,8203 | 0,8083 |
| (AA+A2EH) | 1,78 | 1,62 | 1,61 | 1,78 | 1,62 | 1,63 |
| 2-éthyl hexyl propionate de 2-éthyl hexyle | 11,53 | 11,56 | 11,55 | 11,53 | 11,60 | 11,60 |

Ce test met en évidence la formation de 2-éthyl hexanol résultant de réactions de rétro-craquage dans les conditions de fonctionnement (température, pression, durée) d'une colonne de purification DWC classique.

### Exemple 2 : comparatif

Un mélange réactionnel brut issu de la synthèse par estérification d'acide acrylique avec du 2-éthyl hexanol a été soumis à un traitement de purification à l'aide d'une colonne à partition telle que celle décrite dans l'exemple 1 du document WO 2018/114429.

Une simulation à l'aide du logiciel ASPEN Plus a été réalisée pour un mélange réactionnel ayant la composition massique suivante :
A2EH : 95%
2-éthyl hexanol (E2HOH) : 1,5 %
2-éthyl hexyl propionate de 2-éthyle hexyle (OPO) : 0,76%
Béta hydroxypropionate de 2-éthyl hexyle (HPE2H) : 2,4%
Acétate de 2-éthylhexyle (AC2EH) : 0,22%

La pression de tête de colonne est de 25mm Hg , la perte de charge de la colonne a été fixée à 48 mm Hg.

Le débit de tête est de 5462 kg/h et le débit de pied est de 1200 kg/h.

La colonne comporte 26 étages théoriques au total. La température de pied est de 143°C, la température de tête est de 111°C.

On obtient ainsi un profil de concentration massique (étage 1 : tête de colonne ; étage 26 pied de colonne) pour l'ensemble de la colonne, tel que représenté dans le tableau 2.

**Tableau 2**

| Etage | E2HOH | A2EH | OPO | HPE2H | AC2EH |
|---|---|---|---|---|---|
| 1 | 0,02570981 | 0,97129935 | 3,05E-07 | 2,66E-05 | 0,00290101 |
| 2 | 0,01626731 | 0,98123475 | 7,44E-07 | 5,62E-05 | 0,00237266 |
| 3 | 0,0149886 | 0,98264069 | 1,06E-06 | 7,59E-05 | 0,00222324 |
| 4 | 0,01466844 | 0,9830128 | 1,50E-06 | 0,00010205 | 0,0021429 |
| 5 | 0,01460183 | 0,98308381 | 2,12E-06 | 0,00013669 | 0,00210175 |
| 6 | 0,01460157 | 0,98305565 | 3,00E-06 | 0,00018257 | 0,00208229 |
| 7 | 0,01461954 | 0,98298269 | 4,24E-06 | 0,00024325 | 0,00207468 |
| 8 | 0,01464346 | 0,98287787 | 5,99E-06 | 0,00032342 | 0,00207347 |
| 9 | 0,01467011 | 0,98274114 | 8,47E-06 | 0,00042922 | 0,00207567 |
| 10 | 0,01469858 | 0,98256681 | 1,20E-05 | 0,0005687 | 0,00207964 |
| 11 | 0,01472855 | 0,98234526 | 1,69E-05 | 0,00075238 | 0,00208448 |
| 12 | 0,01475995 | 0,98206281 | 2,39E-05 | 0,00099401 | 0,00208969 |
| 13 | 0,0147928 | 0,9817011 | 3,37E-05 | 0,00131154 | 0,00209502 |
| 14 | 0,01501932 | 0,98108292 | 4,73E-05 | 0,00172142 | 0,00206767 |
| 15 | 0,00644444 | 0,98978944 | 5,20E-05 | 0,00188171 | 0,00176416 |
| 16 | 0,0026873 | 0,99360468 | 5,79E-05 | 0,0020833 | 0,0014903 |
| 17 | 0,00110501 | 0,99513948 | 6,58E-05 | 0,00234971 | 0,00125318 |
| 18 | 0,00045124 | 0,99561167 | 7,67E-05 | 0,0027091 | 0,00105147 |
| 19 | 0,00018362 | 0,99552906 | 9,19E-05 | 0,00319797 | 0,00088107 |
| 20 | 7,46E-05 | 0,99507182 | 0,00011313 | 0,00386534 | 0,00073745 |
| 21 | 3,03E-05 | 0,99426733 | 0,00014302 | 0,00477799 | 0,00061644 |
| 22 | 1,23E-05 | 0,99306099 | 0,00018517 | 0,00602721 | 0,00051444 |
| 23 | 5,02E-06 | 0,99133933 | 0,00024472 | 0,00773784 | 0,00042837 |
| 24 | 2,05E-06 | 0,98893046 | 0,00032893 | 0,01008068 | 0,00035567 |
| 25 | 8,35E-07 | 0,98559199 | 0,00044809 | 0,01328907 | 0,00029417 |
| 26 | 4,88E-07 | 0,9831384 | 0,00053576 | 0,01563413 | 0,00026547 |

Au niveau des plateaux 18-19-20, les compositions sont proches de celles obtenues dans de l'exemple 1 du document WO 2018114429, et la pureté du produit A2EH est de l'ordre de 99,5%.

Cependant, la simulation Aspen ne tient pas compte des phénomènes de rétro-craquage pouvant se produire dans la colonne.

Pour simuler l'effet du rétro-craquage sur la qualité du produit que l'on obtiendrait aux plateaux 19 et 20, un essai de simulation a été réalisé dans les mêmes conditions, mais après avoir ajouté en pied de colonne dans le bouilleur un flux de 1,2 kg/h de 2-éthyl hexanol, soit 1000 ppm par rapport au flux de pied.

On obtient ainsi un profil de concentration massique (étage 1 : tête de colonne ; étage 26 pied de colonne) pour l'ensemble de la colonne, tel que représenté dans le tableau 3.

**Tableau 3**

| Etage | | | | | |
|---|---|---|---|---|---|
| | E2HOH | A2EH | OPO | HPE2H | AC2EH |
| 1 | 0,02599795 | 9,71E-01 | 3,04E-07 | 2,65E-05 | 0,00290011 |
| 2 | 0,01645191 | 9,81E-01 | 7,44E-07 | 5,62E-05 | 0,00237224 |
| 3 | 0,01515807 | 9,82E-01 | 1,05E-06 | 7,59E-05 | 0,00222285 |
| 4 | 0,0148339 | 9,83E-01 | 1,49E-06 | 0,000102 | 0,0021425 |
| 5 | 0,01476637 | 9,83E-01 | 2,12E-06 | 0,00013663 | 0,00210133 |
| 6 | 0,01476604 | 9,83E-01 | 2,99E-06 | 0,00018249 | 0,00208185 |
| 7 | 0,01478418 | 9,83E-01 | 4,24E-06 | 0,00024314 | 0,00207423 |
| 8 | 0,01480835 | 9,83E-01 | 5,99E-06 | 0,00032328 | 0,00207302 |
| 9 | 0,01483529 | 9,83E-01 | 8,47E-06 | 0,00042905 | 0,00207522 |
| 10 | 0,01486406 | 9,82E-01 | 1,20E-05 | 0,00056847 | 0,00207918 |
| 11 | 0,01489436 | 9,82E-01 | 1,69E-05 | 0,00075209 | 0,00208402 |
| 12 | 0,0149261 | 9,82E-01 | 2,39E-05 | 0,00099364 | 0,00208923 |
| 13 | 0,01495931 | 9,82E-01 | 3,37E-05 | 0,00131108 | 0,00209456 |
| 14 | 0,01518837 | 9,81E-01 | 4,72E-05 | 0,00172083 | 0,00206722 |
| 15 | 0,0066295 | 9,90E-01 | 5,19E-05 | 0,00188104 | 0,00176376 |
| 16 | 0,00287779 | 9,93E-01 | 5,79E-05 | 0,00208256 | 0,00148998 |
| 17 | 0,00129682 | 9,95E-01 | 6,58E-05 | 0,00234888 | 0,00125295 |
| 18 | 0,00064303 | 9,95E-01 | 7,67E-05 | 0,00270817 | 0,00105131 |
| 19 | 0,00037506 | 9,95E-01 | 9,19E-05 | 0,0031969 | 0,00088096 |
| 20 | 0,00026564 | 0,9948822 | 0,00011309 | 0,00386409 | 0,00073738 |
| 21 | 0,000221 | 0,99407828 | 0,00014297 | 0,0047765 | 0,0006164 |
| 22 | 0,00020275 | 0,99287252 | 0,00018511 | 0,00602538 | 0,00051442 |
| 23 | 0,0001953 | 0,99115146 | 0,00024464 | 0,00773558 | 0,00042837 |
| 24 | 0,00019229 | 0,98874325 | 0,00032882 | 0,01007783 | 0,00035568 |
| 25 | 0,0001912 | 0,98540548 | 0,00044796 | 0,01328544 | 0,00029419 |
| 26 | 0,00027885 | 0,98286735 | 0,00053549 | 0,0156273 | 0,00026546 |

Au niveau des plateaux 18-19-20, la qualité du produit A2EH pur est modifiée, confirmant l'impact négatif d'une génération de 2-éthyl hexanol sur un produit en soutirage latéral placé à 6-8 plateaux théoriques au-dessus du bouilleur. Dans ces conditions, la pureté du A2EH soutiré au plateau 20 est inférieure à 99,5%.

### Exemple 3 : selon l'invention

Une simulation a été réalisée à l'aide du logiciel ASPEN plus pour un système de purification tel que représenté sur la Figure annexée.

L'alimentation est identique à celle de l'exemple 2 avec un ajout au bouilleur de la section de préfractionnement de 1000 ppm de 2-éthyl hexanol pour simuler le craquage.

Le nombre de plateaux des différentes sections est comme suit :
N1 :12 - N2 : 2-N3 :12-N4 : 8 - N5 : 6

Dans cette configuration, on a obtenu un A2EH d'une pureté supérieure à 99,7% avec une teneur résiduelle en 2-éthyl hexanol de 50 ppm.

Selon l'invention, une paroi séparatrice allant jusqu'en bas de la colonne permet d'éviter que les produits de rétro-craquage tel que le 2-éthyl hexanol polluent le produit soutiré latéralement.

Cet exemple confirme l'importance du positionnement de la paroi séparatrice dans la colonne à partition pour la qualité du produit obtenu en soutirage latéral, en particulier la nécessité d'avoir une paroi séparant la partie basse de la colonne,

### Exemple 4 : Equeutage de l'acrylate de 2-octyle

Cet essai a été réalisé à l'aide d'une colonne Pilote DN 200 mm pour caractériser les phénomènes de rétro-craquage qui peuvent se produire également dans un procédé de fabrication d'acrylate de 2-octyle à partir de 2-octanol et d'acide acrylique.

Les principales impuretés présentes dans l'alimentation de la colonne, dans le pied de la colonne et en tête de celle-ci figurent dans le tableau 4 ci-après.

**Tableau 4**

| Bilan massique, % | Alimentation | Pied de colonne | Tête de colonne | Bilan S/E (%) |
|---|---|---|---|---|
| débit massique (kg/h) | 65 | 24 | 41 | |
| Acide Acrylique | 0,0075 | 0,0265 | 0,025 | 241 |
| Acrylate de 2-octyle | 99,6965 | 99,2427 | 99,7135 | 0 |
| 2-Octanol | 0,0687 | 0,182 | 0,1323 | 119 |
| Octènes | 0,0389 | 0,1625 | 0,1267 | 260 |
| AA+Alcool | 0,1865 | 0,3856 | | -24 |
| Phénol | 0,0001 | 0,0001 | 0,0002 | 63 |
| Acétate de 2-octyle | 0,0018 | 0,0006 | 0,0023 | -7 |

Le bilan entre la sortie et l'entrée S/E permet de vérifier la variation des espèces présentes dans les flux.

Lors de cette distillation, on constate une forte augmentation de la teneur en acide acrylique, en 2-octanol et en octènes, ce qui indique que le mélange réactionnel subit des réactions de craquage dans les conditions de distillation.

## Revendications

1. Procédé de récupération d'un ester (méth)acrylique en C₄-C₁₂ purifié, à partir d'un mélange réactionnel brut obtenu par estérification directe de l'acide (méth)acrylique avec l'alcool en C₄-C₁₂ correspondant, de préférence en présence d'un catalyseur homogène acide,
ledit procédé étant **caractérisé en ce qu'**il est mis en oeuvre à l'aide d'un système de purification comprenant une colonne à partition équipée d'une paroi séparatrice non jointive avec le dôme supérieur de la colonne en partie haute et jointive avec le fond de la colonne en partie basse, et associée en tête à un condenseur unique et en pied à deux bouilleurs, ladite colonne à partition comprenant une section commune de rectification au-dessus de la paroi, une section de préfractionnement comportant l'alimentation de la colonne et une section de soutirage, séparée de la section de préfractionnement par la paroi, comprenant le soutirage de l'ester purifié.

2. Procédé selon la revendication 1, **caractérisé en ce qu'**un flux gazeux est extrait en tête de la section de rectification et recyclé après condensation au moins en partie dans le réacteur d'estérification.

3. Procédé selon la revendication 1 ou 2 caractérisé en qu'un flux est soutiré en pied de la section de préfractionnement et recyclé au moins en partie dans le réacteur d'estérification.

4. Procédé selon l'une quelconque des revendications précédentes **caractérisé en ce qu'**un flux est soutiré en pied de la section de soutirage et recyclé au moins en partie dans la section de préfractionnement de la colonne à partition.

5. Procédé selon l'une quelconque des revendications précédentes **caractérisé en ce qu'**un flux d'ester (méth)acrylique purifié est soutiré latéralement de la section de soutirage en un point situé au-dessus du soutirage de pied de ladite section de soutirage.

6. Procédé selon l'une quelconque des revendications précédentes **caractérisé en ce que** le mélange réactionnel brut est soumis au préalable à une neutralisation avec une base suivie d'une décantation et d'un lavage de la phase organique avant purification.

7. Procédé selon l'une quelconque des revendications précédentes **caractérisé en ce que** la stabilisation du système de purification est réalisée à l'aide d'un inhibiteur de polymérisation unique, de préférence injecté au niveau du condenseur de tête, l'ester (méth)acrylique purifié étant soutiré latéralement de la colonne à partition sous forme d'un flux liquide ou gazeux déjà stabilisé.

8. Procédé selon l'une quelconque des revendications 1 à 6 **caractérisé en ce que** la stabilisation du système de purification est réalisée à l'aide d'un premier inhibiteur de polymérisation, de préférence injecté au niveau du condenseur de tête, l'ester (méth)acrylique purifié étant soutiré latéralement de la colonne à partition sous forme d'un flux gazeux qui, après condensation, est ensuite stabilisé avec un inhibiteur de polymérisation différent du premier inhibiteur.

9. Procédé selon l'une quelconque des revendications précédentes **caractérisé en ce que** le nombre d'étages théoriques de la section de préfractionnement est compris entre 1 et 15.

10. Procédé selon l'une quelconque des revendications précédentes **caractérisé en ce que** le nombre d'étages théoriques de la section de soutirage est compris entre 2 et 15.

11. Procédé selon l'une quelconque des revendications précédentes **caractérisé en ce que** le nombre d'étages théoriques de la section de rectification est compris entre 5 et 15.

12. Procédé selon l'une quelconque des revendications précédentes **caractérisé en ce que** l'ester (méth)acrylique est l'acrylate de 2-éthyl hexyle ou l'acrylate de 2-octyle.

13. Procédé de production d'un ester (méth)acrylique en C₄-C₁₂ de haute pureté, par estérification directe de l'acide (méth)acrylique avec l'alcool en C₄-C₁₂ correspondant, **caractérisé en ce que** le mélange réactionnel brut est soumis au procédé de récupération à l'aide du système de purification tel que défini selon l'une quelconque des revendications précédentes.

14. Utilisation d'un système de purification pour récupérer un ester (méth)acrylique en C₄-C₁₂ purifié, à partir d'un mélange réactionnel brut obtenu par estérification directe de l'acide (méth)acrylique avec l'alcool en C₄-C₁₂ correspondant, ledit système de purification comprenant une colonne à partition équipée d'une paroi séparatrice non jointive avec le dôme supérieur de la colonne en partie haute et jointive avec le fond de la colonne en partie basse, et associée en tête à un condenseur unique et en pied à deux bouilleurs, ladite colonne à partition comprenant une section commune de rectification au-dessus de la paroi, une section de préfractionnement comportant l'alimentation de la colonne et une section de soutirage, séparée de la section de préfractionnement par la paroi, comprenant le soutirage de l'ester purifié.

## Patentansprüche

1. Verfahren zur Gewinnung eines gereinigten C₄-C₁₂-(Meth)Acrylsäureesters aus einer rohen Reaktionsmischung, die durch direkte Veresterung der (Meth)Acrylsäure mit dem entsprechenden C₄-C₁₂-Alkohol vorzugsweise in Gegenwart eines homogenen sauren Katalysators erhalten wird,
wobei das Verfahren **dadurch gekennzeichnet ist, dass** es mittels eines Reinigungssystems durchgeführt wird, das eine Trennwandkolonne umfasst, die mit einer Trennwand ausgestattet ist, die nicht mit dem oberen Dom der Kolonne im oberen Abschnitt verbunden ist und mit dem Boden der Kolonne im unteren Abschnitt verbunden ist und am Kopf einen einzelnen Kühler und am Fuß zwei Erhitzer aufweist, wobei die Trennwandkolonne oberhalb der Wand einen gemeinsamen Rektifizierabschnitt, einen die Zufuhr zur Kolonne umfassenden Vorfraktionierungsabschnitt und einen Entnahmeabschnitt umfasst, der durch die Wand vom Vorfraktionierungsabschnitt getrennt ist, der die Entnahme des gereinigten Esters umfasst.

2. Verfahren nach Anspruch 1, **dadurch gekennzeichnet, dass** am Kopf des Rektifizierabschnitts ein Gasstrom abgenommen und nach einer Kondensation zumindest teilweise in den Veresterungsreaktor zurückgeführt wird.

3. Verfahren nach Anspruch 1 oder 2, **dadurch gekennzeichnet, dass** am Fuß des Vorfraktionierungsabschnitts ein Strom entnommen und zumindest teilweise in den Veresterungsreaktor zurückgeführt wird.

4. Verfahren nach einem der vorhergehenden Ansprüche, **dadurch gekennzeichnet, dass** am Fuß des Entnahmeabschnitts ein Strom entnommen und zumindest teilweise in den Vorfraktionierungsabschnitt der Trennwandkolonne zurückgeführt wird.

5. Verfahren nach einem der vorhergehenden Ansprüche, **dadurch gekennzeichnet, dass** ein Strom von gereinigtem (Meth)Acrylsäureester als Seitenstrom an einer Stelle vom Entnahmeabschnitt entnommen wird, die sich oberhalb der unteren Entnahme des Entnahmeabschnitts befindet.

6. Verfahren nach einem der vorhergehenden Ansprüche, **dadurch gekennzeichnet, dass** die rohe Reaktionsmischung zuvor einer Neutralisation mit einer Base unterzogen wird, gefolgt von einem Dekantieren und einem Waschen der organischen Phase vor der Reinigung.

7. Verfahren nach einem der vorhergehenden Ansprüche, **dadurch gekennzeichnet, dass** die Stabilisierung des Reinigungssystems mittels eines einzelnen Polymerisationsinhibitors erfolgt, der vorzugsweise auf der Höhe des Kopfkühlers eingespritzt wird, wobei der gereinigte (Meth)Acrylsäureester als Seitenstrom in Form eines bereits stabilisierten flüssigen oder gasförmigen Stroms von der Trennwandkolonne entnommen wird.

8. Verfahren nach einem der Ansprüche 1 bis 6, **dadurch gekennzeichnet, dass** die Stabilisierung des Reinigungssystems mittels eines ersten Polymerisationsinhibitors erfolgt, der vorzugsweise auf der Höhe des Kopfkühlers eingespritzt wird, wobei der gereinigte (Meth)Acrylsäureester als Seitenstrom in Form eines gasförmigen Stroms von der Trennwandkolonne entnommen wird, der nach der Kondensation anschließend mit einem Polymerisationsinhibitor stabilisiert wird, der vom ersten Polymerisationsinhibitor verschieden ist.

9. Verfahren nach einem der vorhergehenden Ansprüche, **dadurch gekennzeichnet, dass** die Zahl der theoretischen Böden des Vorfraktionierungsabschnitts zwischen 1 und 15 beträgt.

10. Verfahren nach einem der vorhergehenden Ansprüche, **dadurch gekennzeichnet, dass** die Zahl der theoretischen Böden des Entnahmeabschnitts zwischen 2 und 15 beträgt.

11. Verfahren nach einem der vorhergehenden Ansprüche, **dadurch gekennzeichnet, dass** die Zahl der theoretischen Böden des Rektifizierabschnitts zwischen 5 und 15 beträgt.

12. Verfahren nach einem der vorhergehenden Ansprüche, **dadurch gekennzeichnet, dass** es sich beim (Meth)Acrylsäureester um 2-Ethylhexylacrylat oder 2-Octylacrylat handelt.

13. Verfahren zur Herstellung eines hochreinen C₄-C₁₂-(Meth)Acrylsäureesters durch direkte Veresterung von (Meth)Acrylsäure mit dem entsprechenden C₄-C₁₂-Alkohol, **dadurch gekennzeichnet, dass** die rohe Reaktionsmischung dem Gewinnungsverfahren mittels des Reinigungssystems nach einem der vorhergehenden Ansprüche unterzogen wird.

14. Verwendung eines Reinigungssystems zur Gewinnung eines gereinigten C₄-C₁₂-(Meth)Acrylsäureesters aus einer rohen Reaktionsmischung, die durch direkte Veresterung der (Meth)Acrylsäure mit dem entsprechenden C₄-C₁₂-Alkohol erhalten wird, wobei das Reinigungssystem eine Trennwandkolonne umfasst, die mit einer Trennwand ausgestattet ist, die nicht mit dem oberen Dom der Kolonne im oberen Abschnitt verbunden ist und mit dem Boden der Kolonne im unteren Abschnitt verbunden ist und am Kopf einen einzelnen Kühler und am Fuß zwei Erhitzer aufweist, wobei die Trennwandkolonne oberhalb der Wand einen gemeinsamen Rektifikationsabschnitt, einen die Zufuhr zur Kolonne umfassenden Vorfraktionierungsabschnitt und einen Entnahmeabschnitt umfasst, der durch die Wand vom Vorfraktionierungsabschnitt getrennt ist, der die Entnahme des gereinigten Esters umfasst.

## Claims

1. A process for recovering a purified C₄-C₁₂ (meth)acrylic ester, starting from a crude reaction mixture obtained by direct esterification of the (meth)acrylic acid with the corresponding C₄-C₁₂ alcohol, preferably in the presence of a homogeneous acid catalyst, said process being **characterized in that** it is carried out using a purification system comprising a dividing wall column equipped with a separating wall which is not joined to the upper dome of the column in the top part and is joined to the bottom of the column in the bottom part, and associated at the top with a single condenser and at the bottom with two boilers, said dividing wall column comprising a common rectification section above the wall, a prefractionation section comprising the feed of the column, and a withdrawal section separated from the prefractionation section by the wall and comprising the withdrawal of the purified ester.

2. The process as claimed in claim 1, **characterized in that** a gas stream is extracted at the top of the rectification section and recycled after condensation at least in part into the esterification reactor.

3. The process as claimed in claim 1 or 2, **characterized in that** a stream is withdrawn at the bottom of the prefractionation section and recycled at least in part into the esterification reactor.

4. The process as claimed in any one of the preceding claims, **characterized in that** a stream is withdrawn at the bottom of the withdrawal section and recycled at least in part into the prefractionation section of the dividing wall column.

5. The process as claimed in any one of the preceding claims, **characterized in that** a stream of purified (meth)acrylic ester is withdrawn as a sidestream from the withdrawal section at a point situated above the bottom withdrawal of said withdrawal section.

6. The process as claimed in any one of the preceding claims, **characterized in that** the crude reaction mixture is subjected beforehand to neutralization with a base followed by decantation and washing of the organic phase before purification.

7. The process as claimed in any one of the preceding claims, **characterized in that** the purification system is stabilized using a single polymerization inhibitor, preferably injected at the top condenser, the purified (meth)acrylic ester being withdrawn as a sidestream from the dividing wall column in the form of an already stabilized liquid or gas stream.

8. The process as claimed in any one of claims 1 to 6, **characterized in that** the purification system is stabilized using a first polymerization inhibitor, preferably injected at the top condenser, the purified (meth)acrylic ester being withdrawn as a sidestream from the dividing wall column in the form of a gas stream which, after condensation, is subsequently stabilized with a polymerization inhibitor that is different from the first inhibitor.

9. The process as claimed in any one of the preceding claims, **characterized in that** the number of theoretical stages of the prefractionation section is between 1 and 15.

10. The process as claimed in any one of the preceding claims, **characterized in that** the number of theoretical stages of the withdrawal section is between 2 and 15.

11. The process as claimed in any one of the preceding claims, **characterized in that** the number of theoretical stages of the rectification section is between 5 and 15.

12. The process as claimed in any one of the preceding claims, **characterized in that** the (meth)acrylic ester is 2-ethylhexyl acrylate or 2-octyl acrylate.

13. A process for producing a C₄-C₁₂ (meth)acrylic ester of high purity by direct esterification of the (meth)acrylic acid with the corresponding C₄-C₁₂ alcohol, **characterized in that** the crude reaction mixture is subjected to the recovery process using the purification system as defined as claimed in any one of the preceding claims.

14. The use of a purification system for recovering a purified C₄-C₁₂ (meth)acrylic ester, starting from a crude reaction mixture obtained by direct esterification of the (meth)acrylic acid with the corresponding C₄-C₁₂ alcohol, said purification system comprising a dividing wall column equipped with a separating wall which is not joined to the upper dome of the column in the top part and is joined to the bottom of the column in the bottom part, and associated at the top with a single condenser and at the bottom with two boilers, said dividing wall column comprising a common rectification section above the wall, a prefractionation section comprising the feed of the column, and a withdrawal section separated from the prefractionation section by the wall and comprising the withdrawal of the purified ester.
